# EUROPEAN PATENT APPLICATION

(11) **EP 1 925 332 A1**
(43) Date of publication of application: **28.05.2008**
(21) Application number: 07022403.5
(22) Date of filing: 19.11.2007
(51) Int. Cl.: A61M 25/10, A61M 29/02

(54) **Balloon cover**

(30) Priority: 21.11.2006 JP 2006314901
(71) Applicant: Nipro Corporation, Kita-ku, Osaka-shi, Osaka-fu, 531-8510 (JP)
(72) Inventor: Tanaka, Yuji, Osaka-shi Osaka-fu 531-8510 (JP); Miyagawa, Katsuya, Osaka-shi Osaka-fu 531-8510 (JP); Maeda, Yasushi, Osaka-shi Osaka-fu 531-8510 (JP)
(74) Representative: Banzer, Hans-Jörg

(57) **Abstract**

A balloon cover (1) for contracting a once expanded balloon (4) in a balloon catheter which is provided with a compressible cover (2) as an inner tube to receive the balloon (4) and with an outer sheath (3) as an outer tube to receive the compressible cover (2) is provided. An inner diameter of the compressible cover (2) has a diameter such that the expanded balloon (4) is contracted therein to make a smaller profile, and an inner diameter of the outer sheath (3) is smaller than an outer diameter of said compressible cover (2). The balloon cover (1) comprises a primary reducing function that the compressible cover (2) receives the once expanded balloon (4) therein, and a secondary reducing function that the outer sheath (3) receives the compressible cover (2) and decreases the diameter of the compressible cover (2) including the balloon (4) therein.

## Description

### Technical Field

The present invention relates to a balloon cover applied to a balloon catheter for expansion which is inserted into a stenosed site in a blood vessel, biliary tract, esophagus or the like so as to dilate and treat the stenosed site.

### Background Art

With regard to a medical instrument for treatment of obstruction and stenosis of a blood vessel of a patient, a balloon catheter having a balloon for expansion which is inserted into the blood vessel to expand whereby the blood vessel is dilated has been known. In order to pass the balloon catheter through an inner area of the stenosed blood vessel, it is desirable that the balloon profile, that is, the size of the balloon is as small as possible when it is contracted or folded.

There are also some cases where plural parts of the blood vessel are treated by one operation and, in that case, the balloon once expanded and contracted is used again. At that time, since the balloon was once expanded, it is unable to contract to the initial balloon profile and the balloon catheter is often unable to pass through a lesion site.

Therefore, there are two kinds of covers in the conventional balloon cover used for a balloon catheter; one of which is provided with a balloon which is not yet used and another of which receives the balloon after expansion to make the balloon profile as small as possible. In the cover for receiving the balloon after expansion, one end of the cover is equipped with a trumpet-shaped opening and has such a shape with a slightly larger inner diameter as compared to the cover for the non-used balloon so that the balloon after expansion can be easily received.

That is because, when a balloon is pushed into a cover, the balloon itself is so soft that friction occurs between the balloon and an inner cavity of the cover and, resistance upon insertion becomes big due to the friction and it becomes difficult to insert the balloon into a cover with a small diameter.

Meanwhile, an expansion catheter in which a balloon part is folded small by means of a slit tube and a heat-shrinking tube when manufacturing the expansion catheter, and a method for manufacturing the same have been already proposed (see, for example, Japanese Patent Laid-Open No. 08/098,892 A, pages 1 to 6 and Fig. 6)

During the manufacture of the expansion catheter equipped with the balloon, it is possible to make the balloon profile small by means of various methods. However, it is difficult for a balloon once expanded to be easily contracted to become an initial balloon profile.

In the conventional method where the once-expanded balloon is pushed into a cover having a trumpet-shaped opening with a small diameter, it is difficult to push the balloon into a sufficiently small diameter due to a large resistance upon insertion and, as a result, there is a problem that the balloon catheter is unable to be repeatedly passed through a lesion site.

### Disclosure of the Invention

In order to solve the above-mentioned problems, an object of the present invention is to provide a balloon cover by which the balloon profile after expansion of the balloon is able to be safely or surely made small.

According to the present invention, this object is achieved by a balloon cover as defined in claim 1. The dependent claims define preferred and advantageous embodiments of the invention.

To achieve the above-mentioned object, the present invention relates to a balloon cover for contracting an expanded balloon in a balloon catheter comprising a compressible cover as an inner tube to receive the balloon and an outer sheath as an outer tube to receive said compressible cover, wherein an inner diameter of the compressible cover has a diameter such that the expanded balloon is contracted therein to form a smaller profile, and an inner diameter of the outer sheath has a diameter smaller than an outer diameter of said compressible cover, and wherein the balloon cover comprises a primary reducing function that the compressible cover receives the once expanded balloon therein, and a secondary reducing function that the outer sheath receives the compressible cover therein and reduces the diameter of the compressible cover with the balloon therein.

According to the balloon cover of the present invention having the above constitution, a balloon is able to be forcibly contracted small via two stages comprising the primary reducing function and the secondary reducing function whereby it is now possible to safely and surely reduce the diameter to an extent of a predetermined balloon profile.

According to an embodiment, the compressible cover is provided with a tubular part having a slit in the longitudinal direction and also with an opening end for insertion of the balloon at an end of the tubular part and which is enlarged in a trumpet shape.

One embodiment of the invention is characterized in that the above compressible cover is provided with a tubular part having a linear slit in the longitudinal direction and also with an opening end at one end of the tubular part for insertion of the balloon, the diameter of the opening end being expanded in a trumpet shape.

Another embodiment of the invention is characterized in that the above compressible cover is provided with a tubular part having a spiral slit in the longitudinal direction and also with an opening end for insertion of the balloon where diameter is expanded in a trumpet shape at one end of the tubular part.

Further embodiment of the invention is characterized in that the above compressible cover is provided with a tubular part having a wedged shaped slit in the longitudinal direction and also with an opening end for insertion of the balloon where diameter is expanded in a trumpet shape at one end of the tubular part.

According to the embodiment having the above constitution, a balloon is able to be easily inserted by pushing from an opening end which increases in diameter into a trumpet shape and, when the diameter of the tubular part equipped with the slit is made small, i.e., compressed or squeezed, the once-received balloon is able to be further contracted.

According to the embodiment having the above constitution, a balloon is able to be easily inserted by pushing from the opening end which increases in diameter into a trumpet shape and, as a result of an operation where a compressible cover equipped with a spiral slit is pushed into an outer sheath as if being screwed into the outer sheath, the balloon which is received in the compressible the cover is able to be further contracted.

According to an embodiment said slit is formed at an opposite end to the trumpet-shaped opening end of the tubular part and is longer than a length of the balloon received from the opening end and wherein a cylindrical guide part is provided between the distal end of the slit of the tubular part and the trumpet-shaped opening end.

According to the embodiment having the above constitution, when the balloon is pushed from the trumpet-shaped opening end, it is fixed to a state where the diameter is made small to a predetermined diameter by the tubular guide part and is stabilized to such a state whereby it is possible to suppress the protrusion of the balloon from the slit. In addition, since the full length of the balloon is able to be received in the slit part, the whole balloon is able to be contracted in a united manner.

In accordance with the present invention, a balloon cover for contracting an expanded balloon of a balloon catheter which is able to contract the balloon profile after expansion can be obtained. The balloon cover comprises a compressible cover as an inner tube to receive the balloon and an outer sheath as an outer tube to receive said compressible cover, wherein an inner diameter of the tube of the compressible cover has diameter such that the expanded balloon is contracted to form a smaller profile therein, and an inner diameter of the outer sheath has a diameter smaller than the outer diameter of said compressible cover, and wherein the balloon cover comprises a primary reducing function that the compressible cover receives the once expanded balloon therein, and a secondary reducing function that the outer sheath receives the compressible cover therein and reduces the diameter of the compressible cover including the balloon. The proximal end of the compressible cover is grasped by hand to insert the cover into the outer sheath, or the outer periphery of the proximal end of the cover can have a tapered portion to faciliate insertion into the outer sheath.

### Brief Description of the Drawings

The invention will now be described in more detail with reference to the accompanying drawings, in which:
Fig. 1 shows a balloon cover according to the present invention wherein (a) is an oblique view and (b) is a cross-sectional view.
Fig. 2 shows a compressible cover according to the present invention wherein 2 (a) are illustrative drawings showing a cross section and a side view and 2 (b) is a cross-sectional view showing a compressible cover wherein a balloon is received.
Fig. 3 shows an actual use of the balloon cover according to the present invention wherein 3 (a) is a cross-sectional view of the balloon cover using a long outer sheath, 3(b) is a cross-sectional view of the balloon cover using a short outer sheath and 3(c) is a cross-sectional view of the balloon cover wherein a short outer sheath and a compressible cover with a big diameter part at the proximal end thereof are combined.
Fig. 4 shows modified examples of the compressible cover wherein 4(a) is a front view of a first modified example and 4(b) is a front view of a second modified example.

The embodiments of the balloon cover in the present invention will be illustrated in detail by referring to the drawings.

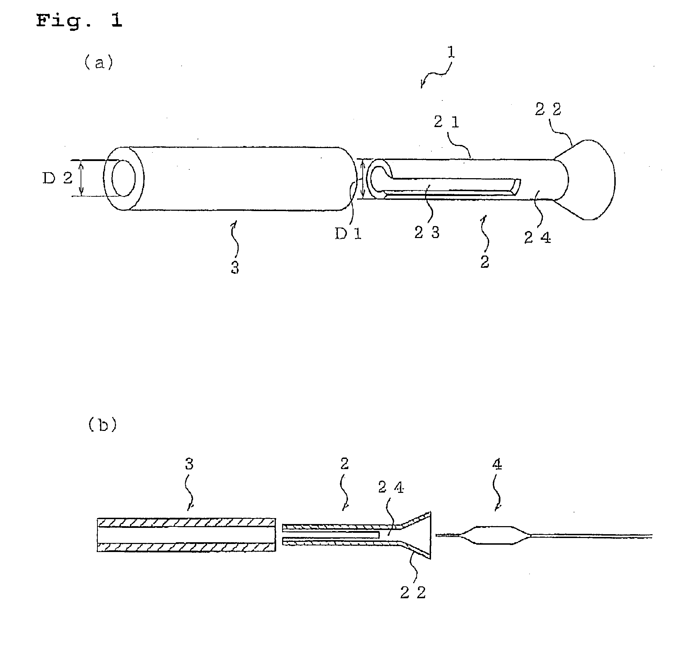

The balloon cover 1 in the present invention is, for example, a cover member by which the diameter of a balloon 4 applied to a balloon catheter for expansion is contracted from an expanded state so that the balloon profile is made small and, and as shown in Fig. 1(a), the balloon cover is provided with a compressible cover 2 and an outer sheath 3. The compressible cover 2 is an inner tube which receives a balloon 4 and the outer sheath 3 is an outer tube which receives the above compressible cover 2.

Thus, the balloon cover 1 is in a double tube-constitution and a compressible cover 2 as the inner tube is preferably made of resin having flexibility. Polyethylene resin, polypropylene resin, etc. are advantageously used for the inner tube. Outer sheath 3 is a tube member for receive the compressible cover 2 by reducing its diameter, and a tube made of resin or metal may be used therefore.

The compressible cover 2 comprises a cylindrical tubular part 21 and a trumpet-shaped opening end 22 at an end of the cylindrical tubular part 21, and a linear slit 23 is provided in an opposite end of the cylindrical tubular part 21 in the longitudinal direction. This slit 23 may be one slit as shown in the drawings or may comprise plural slits which are formed on plural places on the circumference of the tubular part 21.

When the outer diameter D1 of the tubular part 21 in which a slit 23 is provided is made larger than the inner diameter D2 of the outer sheath 3 in which it is to be inserted, it is possible for the outer diameter D1 to be made smaller by an operation where the tubular part 21 is pushed into the outer sheath 3. Thus, it is apparent that the width of the slit 23 is required to be such that it is able to be decreased, or narrowed, when the tubular part 21 is pushed into the outer sheath 3.

The slit 23 is provided at the end of tubular part 21 opposite the trumpet-shaped opening end 22 and extends from the extreme end of the opposite end to a predetermined position in the side of the cylindrical tubular part 21. The slit may .have a length longer than a length of the balloon to be received from the opening end 22 but is not extended to the most distal end of the cylindrical tubular part 21. Thus, a cylindrical guide part 24 (length, L2) is formed between the most distal end of the slit 23 and the trumpet-shaped opening end 22.

Therefore, as shown in Fig. 1(b), when the once-expanded balloon 4 is pushed from the trumpet-shaped opening end 22, the balloon 4 is contracted along the tapered surface of the inlet of the trumpet-shaped opening end 22 and is able to be stably arranged in a predetermined diameter reduced by the cylindrical guide part 24.

As mentioned above, the cylindrical guide part 24 is formed connecting to the trumpet-shaped opening end 22 whereby a gradually contracted balloon can be stably formed. Therefore, even when the balloon 4 is pushed therein, protrusion of the balloon from the slit 23 provided at the proximal end of the cylindrical guide part 24 may be suppressed.

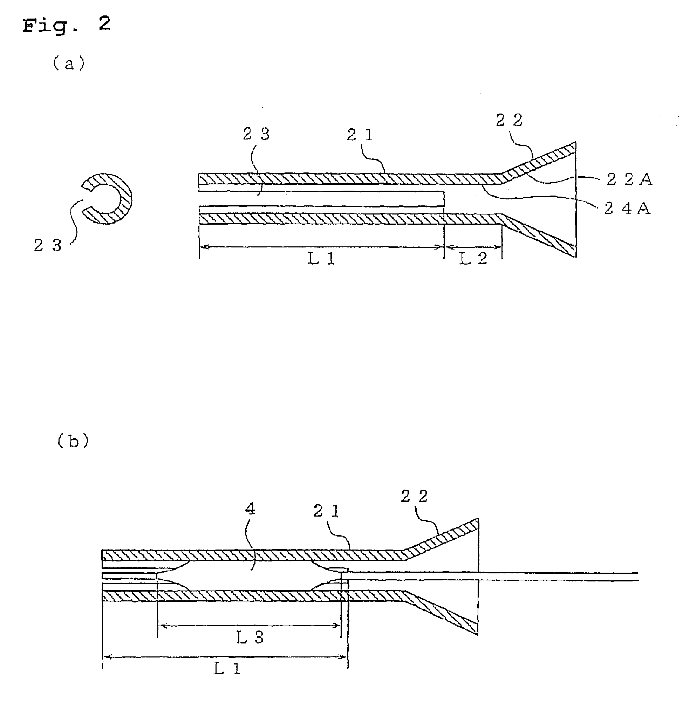

The length L1 of the slit 23 shown in Fig. 2 (a) is preferably longer than the length of the balloon 4 to be received and, as shown in Fig. 2(b), its constitution is that the whole balloon 4 with a length L3 is received in the slit 23.

In the arranged as mentioned above, when the balloon 4 is pushed into the compressible cover 2 from the trumpet-shaped opening end 22, the diameter of the balloon 4 becomes smaller by means of the inner surface 22A of the tapered portion of the trumpet-shaped opening end 22 and the inner surface 24A of the cylindrical guide 24 and the full length of the balloon 4 can be received in the slit 23 in the contracted, or reduced, state. When the diameter of the cylindrical tubular part 21 is reduced so as to make the spaced part, or gap, of the slit 23 narrow, it is apparent that the diameter of the balloon 4 received therein is reduced and the balloon becomes small.

In order to obtain a small balloon profile by reducing the diameter of the once-expanded balloon using the compressible cover 2, it is necessary that the inner diameter of the above compressible cover 2 is made nearly the same as the diameter of the desired balloon profile. In order to reduce the diameter of the compressible cover 2 which receives the balloon 4, an outer sheath 3 having an inner diameter smaller than the outer diameter of the compressible cover 2 is used as mentioned above.

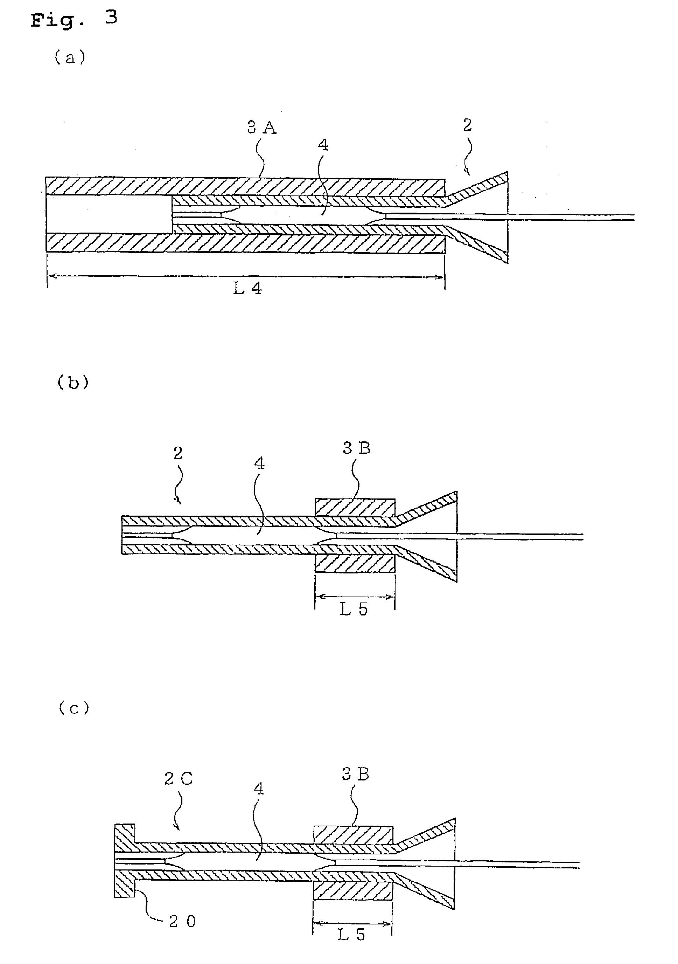

In that case, it is also possible to use an outer sheath 3A having a longer length than the tubular part 21 of the compressible cover 2 as shown in Fig. 3(a) or to use an outer sheath 3B having a shorter length than the tubular part 21 of the compressible cover 2 as shown in Fig. 3(b).

When an outer sheath 3A having a longer length L4 than the tubular part 21 of the compressible cover 2 is used, the entire tubular part 21 of the compressible cover 2 is held in the sheath where the reduced diameter is maintained and, therefore, the sheath 3A can advantageously be applied as an outer sheath which receives the balloon 4 of a material or a shape necessary for a continuous pressurization in a period not shorter than a predetermined time.

When an outer sheath 3B having a shorter length L5 than the tubular part 21 of the compressible cover 2 is used, it is advantageously able to be applied to a balloon 4 which is in a shape and of a material easily compressed, or contracted, by an operation of passing the tubular part 21 of the compressible cover 2 into the sheath. Further, when the compressible cover 2C with a large diameter part 20 at a proximal end thereof and the above-mentioned outer sheath 3B are combined and unified as shown in Fig. 3(c), the two materials are not detached and a balloon cover 1 which is easily handled is provided.

As mentioned above, with the balloon cover 1 of the present invention, the inner diameter of the compressible cover 2 is set to such an extent that the diameter of expanded balloon 4 is reduced to give a small balloon profile and, therefore, when the once expanded balloon 4 is received in the compressible cover 2, the primary reducing function where the outer diameter of the balloon 4 is reduced is achieved. Further, when the compressible cover 2 in a state of having received the balloon 4 is received in the outer sheath 3, the secondary reducing function where the outer diameter of the balloon 4 is further reduced is achieved.

Therefore, it is now possible that the outer diameter of the balloon 4 is forcibly reduced and the predetermined balloon profile is achieved by means of the primary reducing function and the secondary reducing function of the balloon cover 1 of the present invention.

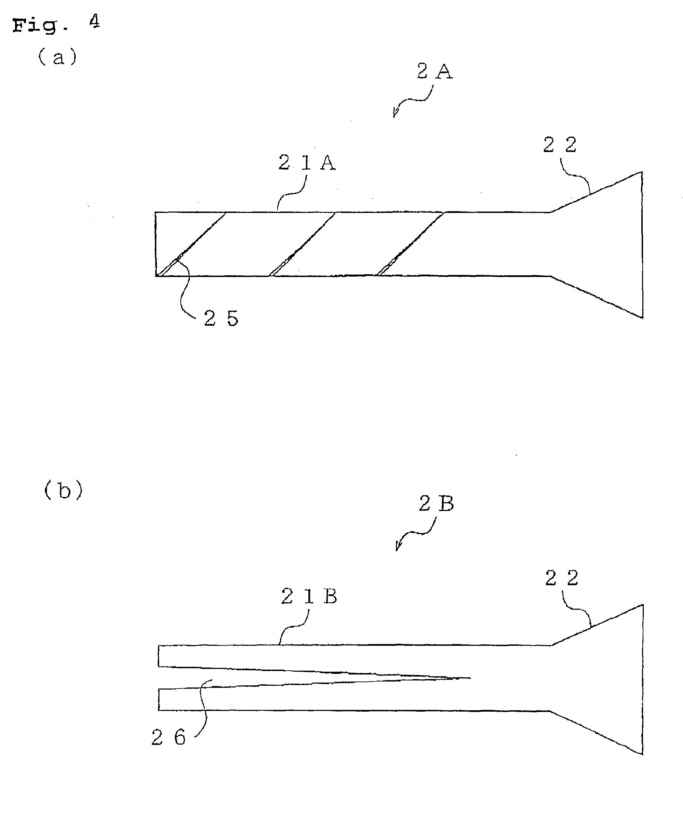

Moreover, the shape of the slit may be not only a slit having a linear opening but also a slit in a spiral or having wedged shape in a longitudinal direction or a slit in other shape may be provided and a first modified example thereof is shown in Fig. 4(a) while a second modified example thereof is shown in Fig. 4(b). The compressible cover 2A shown in the first modified example has a spiral slit 25 in the tubular part 21A. Since the slit 25 is spiral in this constitution, a diameter of the balloon received in the compressible cover 2A is reduced by an operation where the compressible cover 2A is pushed into the outer sheath 3 as if being screwed into the sheath.

Thus, the compressible cover 2A is not straightly pushed therein but is pushed by being screwed thereinto and, therefore, the resistance to pushing in a pushing operation is minimized and reducing of the balloon profile of the balloon 4 is easily conducted.

In the second modified example shown in Fig. 4(b), there is shown a compressible cover 2B equipped with wedge-shaped slits 26 on both sides of the tubular part 21B. When a tubular part 21B having the wedge-shaped slits 26 is pushed into the outer sheath 3 in this constitution, the proximal end side having a big opening width is firstly pushed therein and, therefore, the balloon is easily deformed and pushing is conducted with a small resistance against the pushing. Further, since the wedge-shaped slits 26 are provided on both sides of the tubular part 21B, the balloon 4 is easily compressed in a direction perpendicular to an axial direction of the balloon cover 1 and the cover 1 is advantageously applied when the diameter of balloon 4 having a constitution of compressing in one predetermined direction is to be reduced.

In any of the compressible covers of 2, 2A and 2B, it is common to them that a cylindrical guide part 24 is included between the slit 23, 25, or 26 and the trumpet-shaped opening end 22.

The balloon 4 is manufactured from a substance abundant in flexibility and softness as a raw material such as a polyolefin such as polyethylene and polypropylene, polyvinyl chloride, a thermoplastic elastomer such as a polyurethane and a polyamide elastomer and silicone rubber, latex rubber, etc.. Accordingly, when the balloon 4 is pushed into a cover member with a small diameter so as to reduce the diameter in single sep, there is generally a problem that it is bent or kinked.

However, when the balloon cover 1 according to the present invention is used, due to the compressible action in two stages comprising a primary reducing function in the first stage and a secondary reducing function in the second stage, it is sufficient that the diameter is just reduced to the predetermined balloon profile whereby it is not necessary to forcibly push the balloon into the cover member 1 having a small diameter and the balloon 4 is not bent.

Further, after the balloon 4 is once received in the compressible cover 2, the balloon 4 is inserted into the outer sheath 3 together with the compressible cover 2 whereby, even if the balloon 4 is soft and flexible, the balloon profile is made small safely and surely without kinking.

As mentioned hereinabove, in accordance with the present invention, the balloon cover 1 is made in a constitution achieving a primary reducing function where the once-expanded balloon 4 is received in a compressible cover 2 equipped with a slit and the secondary reducing function where the diameter of the above compressible cover 2 in a state of having received the balloon 4 is made small and the cover 2 is received in the outer sheath at the same time whereby a reducing action in two stages results and it is possible to prepare a balloon cover 1 where the balloon profile after expansion is able to be made small safely and surely.

Further, the slit which is provided in the compressible cover 2 is selected from a linear slit, a spiral slit and a wedged shape slit depending upon the material and shape of the balloon of which the diameter is to be reduced

Still further, since the balloon cover is easily received in and drawn out of the outer sheath and the balloon is easily inserted in and drawn out of the balloon cover, the balloon cover is advantageously used when an operation is carried out using the same balloon repeatedly.

## Claims

1. A balloon cover (1) for contracting a once expanded balloon (4) of a balloon catheter comprising a compressible cover (2; 2C; 2A; 2B) which is an inner tube for receiving the balloon (4) and an outer sheath (3; 3A; 3B) which is an outer tube for receiving said compressible cover (2; 2A; 2B; 2C),
wherein an inner diameter of the compressible cover (2; 2A; 2B; 2C) has a diameter such that the once expanded balloon (4) when received therein is contracted to make a smaller profile, and an inner diameter (D2) of the outer sheath (3; 3A; 3B) is smaller than an outer diameter (D1) of said compressible cover (2; 2A; 2B; 2C), and
wherein the balloon cover (1) comprises a primary reducing function that the compressible cover (2; 2A; 2B; 2C) receives the once expanded balloon (4) therein, and a secondary function that the outer sheath (3; 3A; 3B) receives the compressible cover (2; 2A; 2B; 2C) therein and reduces the diameter (D1) of the compressible cover (2; 2A; 2B; 2C) with the balloon (4) therein.

2. The balloon cover (1) according to claim 1, wherein the compressible cover (2; 2A; 2B; 2C) is provided with a tubular part (21; 21A; 21B) having at a first end a slit (23; 25; 26) extending in the longitudinal direction of the tubular part (21; 21A; 21B) and is also provided with an opening end (22) for insertion of the balloon (4) at an end of the tubular part (21; 21A; 21B) opposite said first end, said opening end (22) being enlarged in a trumpet shape.

3. The balloon cover (1) according to claim 1 or claim 2, wherein the compressible cover (2; 2C) is provided with a tubular part (21) having at a first end a linear slit (23) extending in the longitudinal direction and is also provided with an opening end (22) for insertion of the balloon (4) at an end of the tubular part (21) opposite said first end, said opening end (22) being enlarged in a trumpet shape.

4. The balloon cover (1) according to any of claims 1-3, wherein the above compressible cover (2A) is provided with a tubular part (21A) having at a first end a spiral slit (25) extending in the longitudinal direction and is also provided with an opening end (22) for insertion of the balloon (4) at an end of the tubular part (21A) opposite said first end, said opening end (22) being enlarged in a trumpet shape.

5. The balloon cover (1) according to any of claims 1-4, wherein the above compressible cover (2B) is provided with a tubular part (21B) having at one end a wedge-shaped slit (26) extending in the longitudinal direction and is also provided with an opening end (22) for insertion of the balloon (4) at an end of the tubular part (21B) opposite said first end, said opening end (22) being enlarged in a trumpet shape.

6. The balloon cover (1) according to claim 2, wherein said slit (23; 25; 26) has a length (L1) in the longitudinal direction that is longer than a length (L3) of the once expanded balloon (4) inserted in the opening end (22) and
wherein a cylindrical guide part (24) is provided between the distal end of the slit (23; 25; 26) in the tubular part (21; 21A; 21B) and the trumpet-shaped opening end (22).

7. The balloon cover (1) according to claim 3, wherein said linear slit (23) has a length (L1) in the longitudinal direction that is longer than a length (L3) of the once expanded balloon (4) inserted in the opening end (22) and
wherein a cylindrical guide part (24) is provided between the distal end of the slit (23) in the tubular part (21) and the trumpet-shaped opening end (22).

8. The balloon cover (1) according to claim 4 wherein said spiral slit (25) extends in the longitudinal direction of the tubular part (21A) for a distance that is greater than a length (L3) of the once expanded balloon (4) inserted in the opening end (22) and
wherein a cylindrical guide part (24) is provided between the distal end of the slit (25) in the tubular part (21A) and the trumpet-shaped opening end (22).

9. The balloon cover (1) according to claim 5 wherein said wedge-shaped slit (26) in the longitudinal direction has a length that is longer than a length (L3) of the once expanded balloon (4) inserted in the opening end (22) and
wherein a cylindrical guide part (24) is provided between the distal end of the slit (26) in the tubular part (21B) and the trumpet-shaped opening end (22).

10. The balloon cover (1) according to any of claims 2-9, wherein the outer sheath (3A) has a longer length (L4) than the tubular part (21) of the compressible cover (2).

11. The balloon cover (1) according to any of claims 2-9, wherein the outer sheath (3B) has a shorter length (L5) than the tubular part (21) of the compressible cover (2).
